# EUROPEAN PATENT SPECIFICATION

(11) **EP 0 456 698 B1**
(45) Date of publication and mention of the grant of the patent: **01.07.1998**
(21) Application number: 90902632.0
(22) Date of filing: 08.02.1990
(51) Int. Cl.: C12N 15/75, A61K 39/10

(54) **NOVEL EXPRESSION VECTORS AND METHODS FOR INTRACELLULAR PROTEIN PRODUCTION**
EXPRESSIONSVEKTOREN UND VERFAHREN ZUR HERSTELLUNG INTRAZELLULARER PROTEINE
NOUVEAUX VECTEURS ET PROCEDES D'EXPRESSION POUR LA PRODUCTION DE PROTEINES INTRACELLULAIRES

(30) Priority: 10.02.1989 US 308861
(43) Date of publication of application: 21.11.1991
(73) Proprietor: THE FINNISH NATIONAL PUBLIC HEALTH INSTITUTE, SF-00300 Helsinki (FI)
(72) Inventor: PALVA, Ilkka, SF-00660 Helsinki (FI)
(74) Representative: Bizley, Richard Edward
(86) International application number: FI9000041
(87) International publication number: WO9009448

(56) References cited:
- EP-A- 0 232 229
- EP-A- 0 281 530
- GB-A- 2 091 268
- GB-A- 2 133 408
- EUR. J. Biochem., Vol. 155, 1986 Mervi Sibakov: "High expression of Bacillus licheniformis alfa.amalase with a Bacilluns secretion vector", see page577 page 581.
- JOURNAL OF BACTERIOLOGY, Vol. 162, No. 1, 1985 I. ULMANEN et al: "Transcription and Translation of Foreign Genes in Bacillus subtilis by the Aid of a Secretion Vector", see page 176 - page 182.
- Proc. Natl. Acad. Sci. USA (1982) 79: 5582
- Vaccine (1990) 8: 600
- J. Bacteriol. (1985) 162: 176
- FEMS Microbiol. Lett. (1991) 83: 29

## Description

The present invention relates to recombinant DNA molecules, and methods for producing proteins by said molecules. The invention is particularly concerned with recombinant DNA molecules and methods that lead to high intracellular expression of desired proteins. More specifically the invention is related to the regulation sequence and truncated, non-functional excretion signal sequences of the α-amylase gene of B. amyloliguefaciens, to which DNA encoding a desired structural protein may be joined. It has been found that, for certain structural proteins, Bacillus expression may be surprisingly enhanced, and is to be desired, when intracellular production is achieved according to this aspect of the invention. As will be described in the following, these recombinant DNA molecules can be used, for example, to intensify the α-amylase production in Bacillus strain bacteria, and their modifications to produce any desired homologous or heterologous protein or peptide in Bacillus strain bacteria.

Recent developments in molecular biology have created new possibilities for protein production in bacteria by recombinant DNA techniques.

Even though experiments of this type have been carried out using E. coli plasmid or virus DNA molecules replicating in it as host bacterium, the use of Bacillus strain bacteria as hosts is only beginning (Gryczan et al., Molecular General Genet. 117:459-467 (1978); Keggins et al., Proc. Natl. Acad. Sci. USA 75:1423-1427 (1978); Yoneda et al., Biochem. Biophys. Res. Commun. 91:1556-1564 (1979)).

Palva et al., Proc. Natl. Acad. Sci. USA 79:5582-5586 (1982) discloses use of a vector comprising an incomplete α-amylase signal peptide sequence lacking the six carboxyterminal amino acid residues fused to the E.coli β-lacktamase gene. Expression of such a vector in a host Bacillus cell does not result in secretion of the fused β-lactamase, which instead remained unprocessed and membrane-associated.

GB-A-2133 408 (Palva) discloses a method for improving the production of proteins excreted by recombinant Bacillus bacteria. The recombinant bacteria are characterised by DNA which codes for exoenzymes excreted by the bacteria and which is present in the cell in tens of copies.

GB-A-2091 268 (Palva) concerns recombinant DNA molecules and methods for protein production by Bacillus bacteria. A structural gene of interest is inserted into a bacterial plamid in place of a gene encoding a bacterial exoenzyme. The plasmid contains an excretion signal so that on transformation and expression host cells excrete the desired protein.

Sibakov M. (1986) Eur. J. Biochem. 155:577-581 teaches the construction of a Bacillus amyloliquefaciens α-amylase-based expression/secretion vector carrying the α-amylase gene from B. licheniformis rather than the gene from B. amyloliquefaciens. The vectors comprise an intact B. amyloliquefaciens signal sequence. The recombinant plasmids were stably maintained in B. subtilis and α-amylase activity rose several hundred times from the level of the donor.

Ulmanen I. et al., (1985) Journal of Bacteriology 162: 176-182 reports on research into the transcription and translation of foreign genes in Bacillus subtilis using-a secretion/expression vector. The paper compares the expression of TEM-β-lactamase, Semliki Forest virus glycoprotein EI and B. amyloliquefaciens α-amylase when expressed in B. subtilis by a secretion/expression vector.

The present invention provides a recombinant DNA molecule comprising:-
(a) the regulation sequence of the α-amylase gene of Bacillus amyloliquefaciens;
(b) DNA encoding a non-functional portion of the signal sequence of the a-amylase gene of Bacillus amyloliquefaciens, said signal sequence portion selected from sequences comprising at least the N-terminal initiation methionine (Met) and the next six adjacent N-terminal amino acids of the wild type of said signal sequence but excluding sequences lacking up to the C-terminal last six consecutive amino acids;
(c) DNA encoding a desired homologous or heterologous amino acid sequence;
wherein (c) is in phase with (a) and (b) and said signal sequence portion does not function to secrete said desired amino acid sequence, with the result that the product of expression of (c) is located intracellularly.

A preferred non-functional portion of the signal sequence may comprise amino acids encoded by ATG ATT CAA AAA CGA AAG CGG, ATG ATT CAA AAA CGA AAG CGG AAT TCC, ATG ATT CAA AAA CGA AAG CGG AAT TCG GAA GCT T, or ATG ATT CAA AAA CGA AAG CGG AAT TTA AGC TT.

The DNA encoding amino acids of a desired protein or polypeptide may be DNA encoding chloramphenicol acetyl transferase or DNA encoding pertussis toxin subunits.

The invention also provides a vector comprising any one of the recombinant DNA molecules referred to above.

The invention further includes a method for obtaining intracellular expression of a desired homologous or heterologous protein, or polypeptide, in a Bacillus host, comprising
(a) transforming a desired Bacillus host with a recombinant DNA molecule comprising (1) the regulation sequence of the a-amylase gene of Bacillus amyloliquefaciens, (2) DNA encoding a non-functional signal sequence of the a-amylase gene of Bacillus amyloliquefaciens wherein said DNA comprises DNA encoding at least the N-terminal initiation methionine (Met) and the next six adjacent N-terminal amino acids of the wild type of said signal sequence, but not DNA encoding the seven C-terminal amino acids of the wild type of said signal sequence, and (3) DNA encoding amino acids of a desired protein or polypeptide wherein said DNA encoding said desired protein or polypeptide is downstream from and in phase with said regulation sequence and said DNA encoding said non-functional signal sequence, and
(b) culturing the Bacillus host of step (a) under conditions allowing intracellular expression of said desired homologous or heterologous protein or polypeptide.

The invention thereby includes the use of a vector as defined above for obtaining intracellular expression of a desired homologous or heterologous protein, polypeptide, or peptide in a Bacillus host.

A further aspect of the invention provides a Bacillus host incorporating a vector as hereinbefore defined and the use of said Bacillus host for obtaining intracellular expression of a desired homologous or heterologous protein, polypeptide, or peptide.

The invention will now be described in more detail with reference to drawings in which:
Figure 1 is a schematic illustration of the plasmid pKTH 10 and the general structure of the obtained recombinant DNA molecule;
Figure 2 is a representation of the nucleotide sequence (shown in the 5' - 3' direction) of bases 1 - 480 of the α-amylase gene, showing the ClaI and EcoRI restriction sites, and in which the cleavage site of the signal sequence is indicated by an arrow;

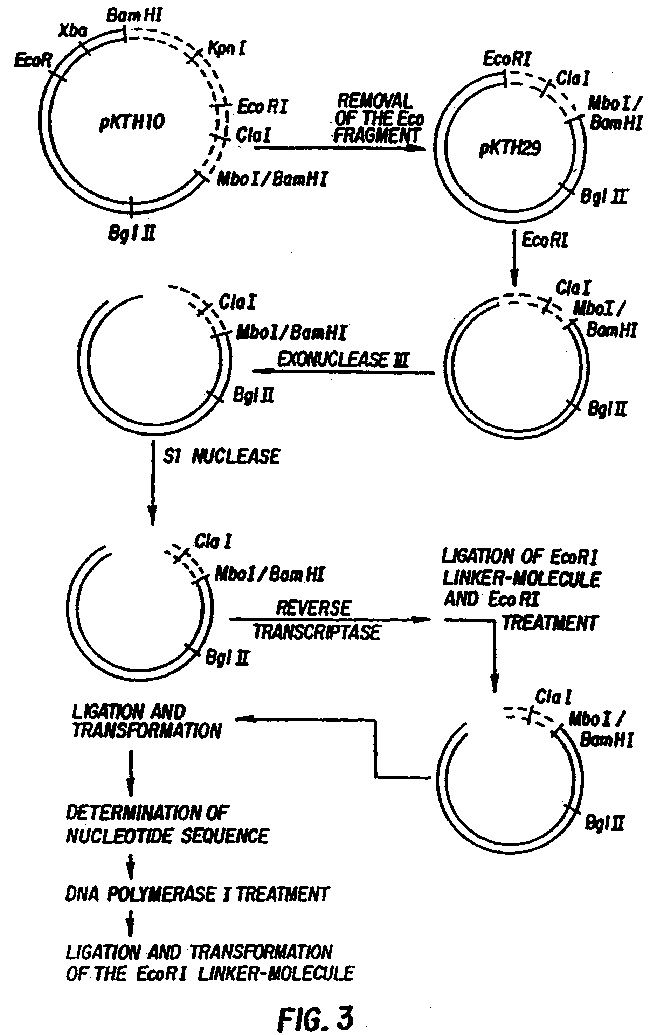

Figure 3 is a flow sheet illustrating the preparation of the recombinant DNA molecule of the present invention containing the regulation and excretion signals of the Bacillus strain α-amylase gene; and
Figure 4 shows the nucleotide sequence of Bacillus expression vectors pKTH 39, pKTH '781 and pKTH 1784, having an α-amylase promoter Palva et al., (1981)) and a truncated α-amylase signal sequence.

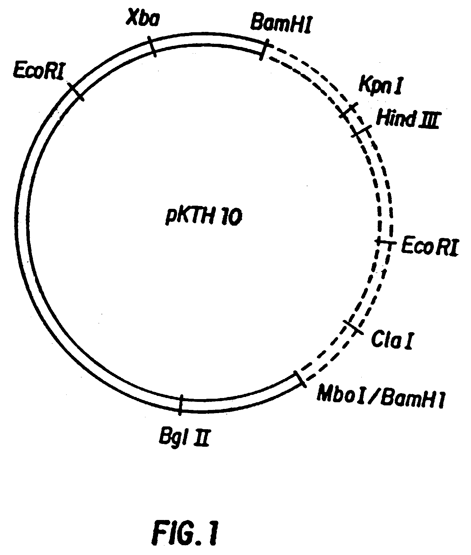

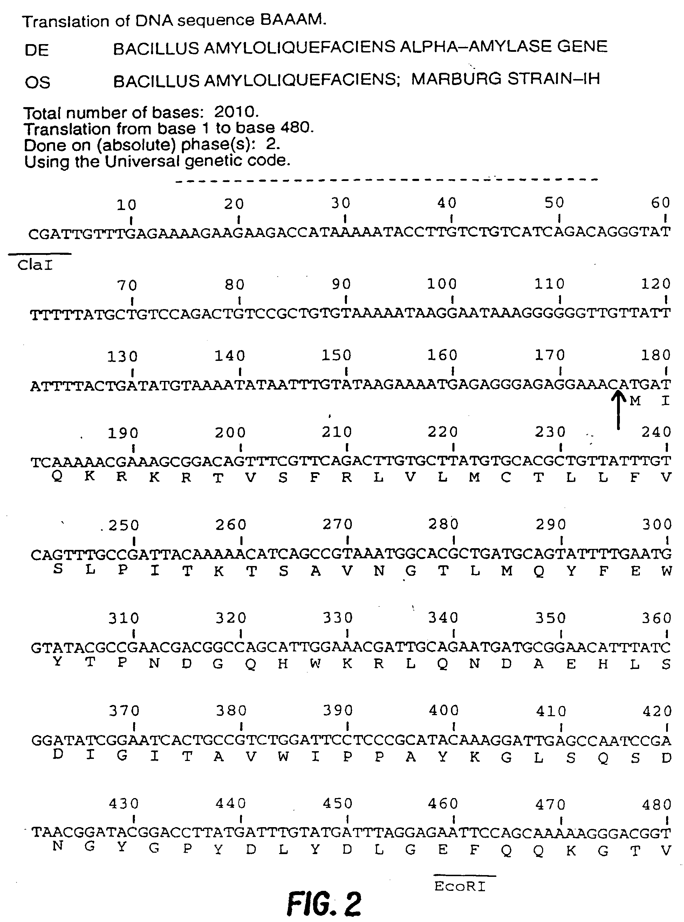

The recombinant DNA molecule is isolated from the B. subtilis strain, and characterized by restriction enzymes and definition of the base order. Figure 1 shows the pKTH 10 of the obtained recombinant DNA molecule, the exclusive restriction enzyme cleavage sites in the α-amylase gene or its regulation signal, and the general structure of the recombinant DNA molecule. Figure 2 shows part of the α-amylase gene base order starting at the cleavage site of the restriction enzyme EcoRI.

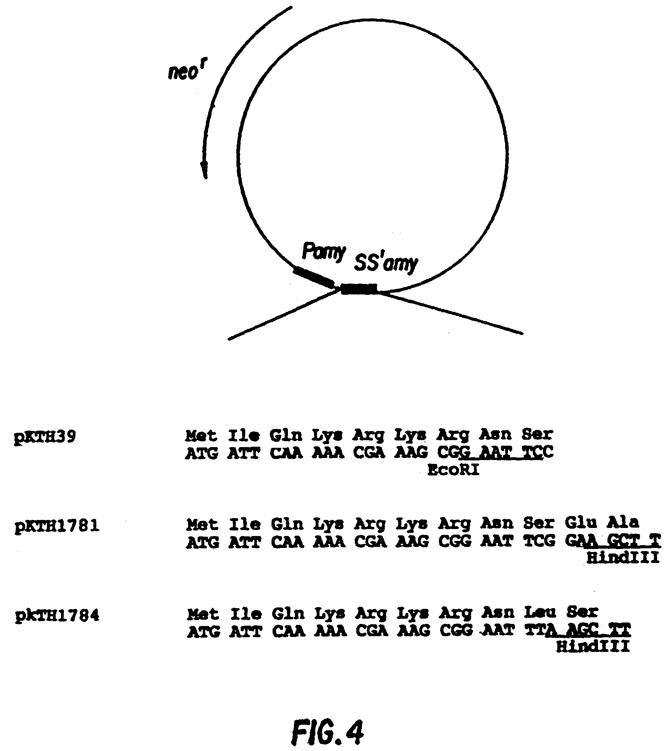

The recombinant DNA molecule concerned in this invention consists of the regulation and truncated excretion signals of the Bacillus strain α-amylase gene, the gene of any protein to be joined at the end of the truncated excretion signal of the α-amylase gene, which results in the desired protein being produced intracellularly in the Bacillus strain bacterium. Recovery can be accomplished by well known methods. The preparation of this recombinant DNA molecule is shown in Figure 4. Thus, by "regulation sequence and truncated nonfunctional signal sequence" of the α-amylase gene of B. amyloliquefaciens is meant the regulation sequence and at least the N-terminal initiation methionine (met) and the terminal amino acids of the wild type of the signal sequence but excluding sequences lacking up to the C-terminal last six consecutive amino acids.

"Substantial part thereof" used herein in reference to a homologous or heterologous protein or peptide or to the gene encoding such protein or peptide is meant to include the "fragments," "variants," "analogs," or "chemical derivatives" of a molecule. A "fragment" of a molecule, such as any of the cDNA sequences of the present invention, is meant to refer to any nucleotide subset of the molecule. A "variant" of such molecule is meant to refer to a naturally occurring molecule substantially similar to either the entire molecule, or a fragment thereof. An "analog" of a molecule is meant to refer to a non-natural molecule substantially similar to either the entire molecule or a fragment thereof.

A molecule is said to be "substantially similar" to another molecule if the sequence of amino acids in both molecules is substantially the same. Substantially similar amino acid molecules will possess a similar biological activity. Thus, provided that two molecules possess a similar activity, they are considered variants as that term is used herein even if one of the molecules contains additional amino acid residues not found in the other, or if the sequence of amino acid residues is not identical. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed, for example, in Remington's Pharmaceutical Sciences, 16th ed., Mack Publishing Co., Easton, Penn. (1980). A "functional derivative" of a gene according to the present invention is meant to include "fragments," "variants," or "analogues" of the gene, which may be "substantially similar" in nucleotide sequence, and which encode a molecule possessing similar activity.

Generally, in preparing the recombinant DNA molecules of the present invention, most of the α-amylase structural gene is first removed by EcoRI restriction enzyme treatment from the recombinant DNA molecule containing the α-amylase gene. The obtained DNA molecule is cleaved by the restriction enzyme and shortened by exonuclease III and S1 nuclease to remove the remaining α-amylase structure gene.
Similarly, the signal sequehce is truncated, whereafter it is secured by a reverse transcriptase enzyme, such that the ends of the molecule are double-stranded. A DNA linker molecule containing the EcoRI cleavage site is then joined to'the cleaved and shortened molecule. The location of the DNA linker in the recombinant DNA molecule is determined by defining the DNA base order at the joining site.

With this method, at the resulting restriction enzyme cleavage site of the DNA linker molecule it is possible to join the structural gene of any other homologous or heterologous protein or peptide, or a substantial part thereof. For example, the β-lactamase of E.coli the DNA sequence (or part of it) of any α, β or γ interferon, chloramphenicol acetyl transferase and any of the subunits of the pertussis toxin are suitable proteins and peptides. The protein or peptide coded by the joined gene will then be produced intracellularly, in the Bacillus strain bacterium by the aid of the regulation and truncated excretion signals of the α-amylase gene. Intracellularly produced protein or peptide may be recovered from the Bacillus host by well known methods.

The choice of particular homologous or heterologous proteins or peptides for intracellular production in accordance with the present invention will be made by those of skill without undue experimentation, with regard to well known principles and establish empirical methods. In Bacillus hosts, cytoplasmic proteins, proteins which are very hydrophobic, and proteins which exhibit strong protein-protein interactions are poor candidates for secretion. Such proteins generally will be good candidates for intracellular production by means of the invention.

Thus, proteins which are not normally secreted in their homologous host cells will be good candidates for intracellular production in Bacillus employing the regulation and truncated non-functional secretion signal sequence of the present invention.

### Removal of the EcoRI fragment from plasmid pKTH 10

The plasmid pKTH 10 was cleaved at the cleavage site EcoRI (Fig. 1). The obtained DNA sequences (about 1 µl T₄-DNA ligase (2 Weiss units) was added. The ligation solution was incubated for 3 hours at 23°C, whereafter the competent B. subtilis IHO 6064 strain was transformed by it in a manner described previously (Anagnostopoulos et al., J. Bcteriol. 81:741-746 (1961)). The recombinant DNA molecules prepared by ligation as described above, were mixed with the competent host bacteria, and the mixture was kept for 30 min at 37°C. The mixture was then spread on bacterium plates with kanamycin antibiotics to prevent the growth of all those bacteria that had not received a plasmid. The cells were spread on bacterium plates containing kanamycin and grown overnight at 37°C. An α-amylase-negative colony was screened from the obtained transformants by the I-KI method (J. Bacteriol. 119:416-424 (1974)) using starch plates, and a plasmid was isolated from the colony in a manner described ealier (Gryczan et al., J. Bacteriol. 134:318-329 (1978)). The missing EcoRI - KpnI - HindIII - EcoRI fragment in the obtained plasmid preparate pKTH 29 was controlled by gel electrophoresis.

### Shortening of plasmid pKTH 29 by exonuclease treatment

The plasmid pKTH 29 (100 µl, 500 µg/ml) was cleaved by the restriction enzyme EcoRI. After this treatment, 0.5 µl 1 M dithiothreitol and 10 µl exonuclease III (0.25 units, Biolabs) were added to the solution. The solution was incubated for 1 - 3 minutes at 37°C, and the reaction was stopped in a 70°C waterbath. The DNA was precipitated from the solution by ethanol and dissolved in a 0.3 M NaCl - 0.03 M sodium acetate - 3 mM ZnCl₂ buffer (pH 4.5). Ten µl Sl-nuclease (25 units/ml, Boehringer Mannheim) was added and the solution was incubated for 30 minutes at 37°C and for 10 min at 4°C. After the incubations, the preparate was extracted with phenol, the phenol was removed by ether extraction, and the DNA was precipitated by ethanol. The dried DNA was dissolved into 40 µl 10 mM Tris HCl - 1 mM EDTA buffer (pH 8.0), and 10 µl 150 mM Tris - 180 mM KCl - 40 mM MgCl₂ - 3.0 mM dithiothreitol buffer (pH 8.3), 5 µl dNTP mixture, in which to each nucleotide-tri-phosphate 10 mM of the solution was mixed in equimolar ratio, and 2 µl reverse transcriptase enzyme (Beard, 13 units/µl) were added. The solution was incubated for 30 minutes at 37°C and the reaction was stopped by incubation at 65°C for 7 minutes. The DNA was purified by preparative agarose electrophoresis (LTG, Low Gelling Temperature), and the plasmid zones that had been dyed with ethidium bromide were cut off from the gel. The DNA was extracted from the agarose by phenol at 65°C, the phenol extraction was repeated at 20°C, and the phenol was removed by ether extraction. The DNA was precipitated by ethanol, and the precipitate was washed with 70 % ethanol and dried.

### Phosphorylation of EcoRI linker molecule its combination to the plasmid

Five µl ³²P γ-ATP (10 mCi/ml, 3000 Ci/mol), 1.7 µl 600 mM Tris HCl - 66 mM MgCl₂ - 100 mM dithiothreitol buffer (pH 8.0) and 0.5 µl T₄-polynucleotide kinase were added to 10 µl EcoRI linker molecule solution (EcoRI linker, Collaborative Research, 50 µg/ml). The solution was incubated for 30 minutes at 37°C, whereafter 5 µl 10 mM ATP was added, and the incubation was continued for 30 min at 37°C. The dried pKTH 29 preparate that had been treated with exonuclease, was dissolved into 5 µl of the solution containing phosphorylated EcoRI-linker-molecule described above. 0.5 µl 10 mM ATP, 0.5µl 1 mM spermidine and 0.5 µl T₄-DNA-ligase (2 Weiss units) were added to the solution. The solution was incubated for 3 hours at 23°C, whereafter it was diluted to 20µl in 40 mM Tris HCl - 100 mM NaCl - 10 mM NgCl₂ buffer (pH 7.6). Fifteen units of EcoRI enzyme (8iolabs) were added, and the solution was incubated for 12 hours at 37°C. The reaction was stopped by incubation at 65°C for 10 minutes. The preparate treated with EcoRI was gelfiltered through a 1 ml Sepharose 4B column. Two mM Tris - HCl - 0.1 mN EDTA (pH 7.5) was used as elution buffer in the filtering. The filtrate was harvested in 35 µl fractions, and the fractions containing plasmid were identified by their radioactivity, collected and dried. The dried DNA was dissolved into 20 µl 66 mM Tris HCl - 6.6 mM MgCl₂-10 mM dithiothreitol buffer (pH 8.0), and 1.5 µl 10 mM ATP and 0.3 µl T₄-DNA-ligase were added. The solution was incubated for 3 hours at 23°C, whereafter the competent B. subtilis IHO 6064 strain was transformed by the plasmid preparate, and the cells were cultivated on bacterium plates containing kanamycin.

The plasmids were isolated from the transformants by a method described earlier (Gryczan et al., J. Bacteriol. 134:318-329 (1978)), and the plasmids were first characterized by gel electrophoresis, whereafter their DNA base sequence at both ends of the EcoRI linker molecule was determined. In this way, plasmids pKTH 38 and pKTH 39 were obtained from the plasmid pKTH 29.

In the plasmid pKTH 38, the EcoRI linker molecule is located 90 nucleotide pairs after the cleavage site of the excretion signal in the area of the α-amylase structural gene. In the plasmid pKTH 39, the EcoRI linker molecule is located 16 nucleotide pairs after the initiation methionine of the α-amylase gene in the area of the signal sequence.

In order to join the linker molecule at the joining site of the excretion signal or in the immediate vicinity thereof, the plasmid pKTH 38 was cleaved with EcoRI. Three portions of 10 µg of the cleaved plasmid were each suspended in 115 µl 20 mM Tris, 600 mM NaCl, 12 mM MgCl₂, 12 mM CuCl₂, 1 mM EDTA buffer (pH 8.1). Ten µl BAL-31 enzyme (Bethesda Research Laboratories, BRL, 40 U/ml) were added to each plasmid portion, and the tubes were incubated for 5, 6 and 7 minutes in a water bath of 30°C. The reaction was stopped by adding 0.5 M EDTA, pH 8.0, so as to obtain a final concentration of 12 mM. The DNA portions treated with BAL-31 were combined, extracted twice with phenol and precipitated with ethanol. The ethanol precipitate was suspended in 75 µl 63 mM Tris, 6.3 mM MgCl₂ buffer (pH 8.0), and to the solution were added 5 µl 1 mM dATP, 1 mM dGTP, 1 mM dCTP, and 1 mM dTTP, and finally 5 µl T4 polymerase (PL-Biochemicals, 5 U/µl). The solution was incubated for 80 minutes at 11°C. The reaction was stopped by adding 0.5 EDTA as above, and the solution was extracted with phenol and the DNA was precipitated with ethanol. The ethanol precipitate was dissolved in 250 µl 10 mM Tris, 1 mM EDTA buffer (pH 8.0). To 55 µl of this solution were added 50 µl phosphorylated Hind III linker molecule (BRL, 75 pmol), 5 µl 660 mM Tris, 100 mM MgCl₂, 50 mM dithiothreitol buffer (pH 7.5), and 10 µl T4 DNA ligase (BRL, 2 U/µl). The mixture was incubated for 15 hours at 15°C and for 10 minutes at 65°C. The DNA was precipitated by adding isopropanol, the DNA precipitate was washed with 70% ethanol and, after drying in vacuo, suspended in 100 µl 10 mM Tris, 50 mM NaCl, 5 mM MgCl, 5 mM dithiothreitol buffer (pH 8.0). Three µl of Hind III restriction enzyme (BRL, 10 U/µl) were added to the suspension, and the solution was incubated for 4 hours at 37°C and for 10 minutes at 65°C. The DNA was purified by electrophoresis in 0.8% LGT agarose gel (Marine Colloids Inc.), at 30 V, for 15 hours. The linear plasmid zone was cut off from the gel, and the DNA was extracted at 65°C with phenol and was precipitated with ethanol. The ethanol precipitate was dissolved in 35 µl 66 mM Tris, 10 mM MgCl, 5 mM dithiothreitol buffer (pH 7.5) to which was added 1.5 µl 10 mM rATP and 1.5 µl T4 DNA ligase (BRL, 2 U/µl). The mixture was incubated for 3 hours at 22°C and transformed into the competent B. subtilis IHO 6135 strain, and the cells were cultivated on nutrient medium plates containing kanamycin. The plasmids were isolated from the transformants according to a method described earlier, and the location of the Hind III linker molecule in the plasmids was determined by means of DNA sequencing. In this way a series of plasmids was obtained in which the Hind III linker molecule is located immediately after the excretion signal or in different positions after the cleavage site of the excretion signal in the area of the α-amylase structure gene.

With respect to the plasmid pKTH 39, in order to join the linker molecule at the appropriate site on the signal sequence, an analogous approach to that described above for the plasmid pKTH 38 is employed. In using the plasmid pKTH 39, which contains the truncated signal sequence of the α-amylase gene of B. amyloliquefaciens, the desired structural gene can thus be inserted either at the ECoRI site, or at the same location using an in vitro modified site as described, for example, in Example 3, or at any location intermediate between the initiation codon (-met) and the EcoRI site, by well known methods. The creation of a new joint site at the desired location may be accomplished by methods known to those of skill, including but not limited to site directed in vitro mutagenesis, polymerase chain reaction (PCR), or by synthesizing in vitro the required promoter fragment, all of which may be accomplished with the exercise of routine skill with an appreciation of the teaching of the present invention.

In addition to the plasmid pKTH 39, the above-described expression unit of the α-amylase gene can be incorporated into any number of suitable Bacillus replicative vectors. Alternatively, it may be integrated into the chromosome of host Bacillus in multiple copies. According to either chosen method, the expression unit of the present invention is suitable for the intracellular expression of a wide variety of structural genes in Bacillus hosts.

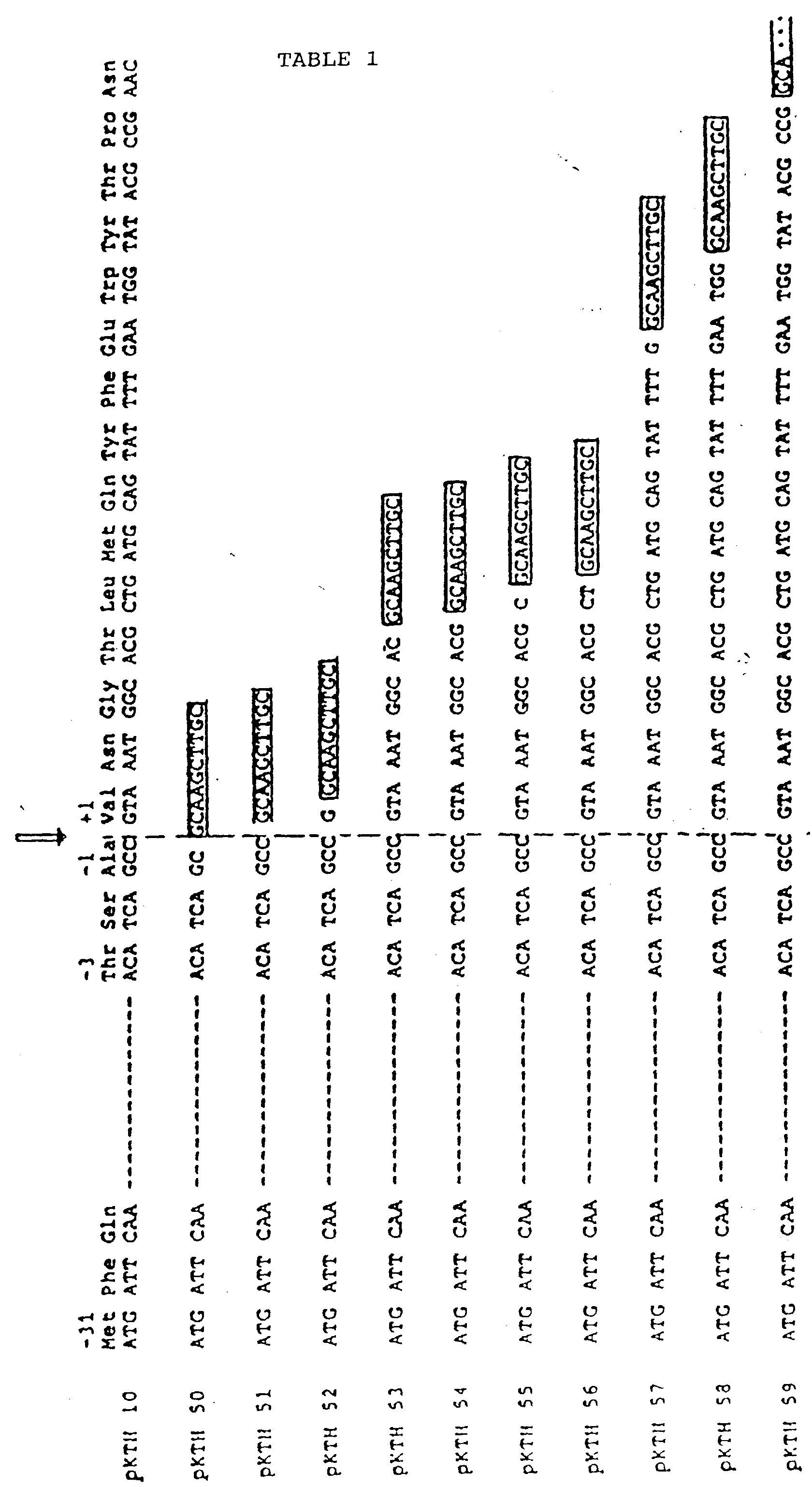

The DNA sequence coding for the amino acids of any desired protein can be joined to the cleavage sites formed by these Hind III linker molecules whereby, as appears from the above Table 1, a bacterium of the Bacillus strain will produce and excrete said protein on its substrate.

A wide variety of proteins may be produced as illustrated by the following listing, presented by general categories:

A. Antigenic proteins of microbes and protozoa: Capsule, outer membrane and Fimbria proteins from the following sources: Bacteroides fragilis, Fusobacterium spp., Bordetella pertussis, Haemophilus influenzae, Yersinia enterocolitica, Yersinia pestis, Branhamella catarrhalis, Escherichia coli, Klebsiella pneumonia, Vibrio cholerae, Proteus mirabilis, Pseudomonas aeruginosa, Serratia marcescens, Legionella pneumophila, Neisseria gonorrhoeae, Neisseria meningitidis, Salmonella typhimurium, Salmonella typhi, Salmonella paratyphi B,Mycobacterium tyberculosis, Chlamydia trachomatis, and Shigella spp.

Protein toxins produced by the following bacteria: Staphylococcus aureus, Pseudomonas aeruginosa, Clostridium spp., Escherichia coli, Yersinia pestis, Vibrio cholerae, Bordetella pertussis, M-protein of the Streptococcus pyogenes bacterium, excreted enzymes of Streptococcus mutans.

Surface proteins of the following organisms (in all phases of development): Plasmodium spp., Toxoplasma spp., Leishmania spp., Schistosoma spp., Trypanosoma spp.

Membrane proteins of the following microorganisms: Mycoplasma pneumoniae, Mycoplasma hominis, contagious protein of Streptococcus spp., and contagious protein of Staphylococcus aureus.

B. Antigen proteins of viruses: HA and NA proteins of myxoviruses (influenza A H1-H12, influenza B, influenza C); HN and F proteins of paramyxoviruses (parainfluenze 1-4, Newcastle disease virus, Measles virus, Respiratory syncytial virus, Parotitis virus, Distemper virus); G protein of Rabies virus; E1 and E2 proteins of alfaviruses (Chikungunya, Western, Eastern, Venezuelan equine encephalitis virus, O'nyong-nyong virus, Semliki Forest virus, Sindbis virus); V1 and V3 proteins of flavin viruses (Denque 1-4, Japanese encephalitis virus, Mite encephalitis viruses, Murray Valley encephalitis virus, Kyasanur Forest disease virus, Looping ill virus, Omsk hemorrhagic fever virus); surface proteins of German measles virus; surface proteins of Hog Cholera virus; surface proteins of Equine arthritis virus; G1 and G2 proteins of Bunya viruses (Rift Valley fever virus, Crimean hemorrhagic fever virus, California encephalitis virus, Phlebotomus fever virus); G1 and G2 proteins of arena viruses (Lassa fever virus, Lymphatic chorion meningitis virus); proteins V1 - V4 of picorna viruses (polio 1 - 3, Coxsackie A viruses 1-24, Coxsackie B viruses 1-6, ECHO viruses 1 - 8, 11 - 34, Hepatitis A virus, Human rhino viruses 1 - 113), surface proteins of rota viruses; surface proteins of herpes viruses (HSV 1, 2, Cytomegalo virus, Epstein-Barr virus, Equine abortion virus); VP1 - VP3 proteins of papova viruses (BK virus, Human wart virus); proteins of parvo viruses (mink enteritis virus, Bovine parvo virus, Feline parvo virus, Procine parvo virus); structure proteins of Human hepatitis B virus; surface proteins of Ebola and Marburg viruses; and Hexone, pentone and fiber proteins of adeno viruses, (Human adeno viruses 1 - 33).

### C. Industrial enzymes:

| | |
|---|---|
| alpha-amylase | (B. subtilis, malt, A. oryzae) |
| Amino acid acylase | (Bacillus spp.) |
| Amyloglucosidase | (A. niger, Rhizopus sp.) |
| Bromelain | (Ananas) |
| Phisine | (Fig) |
| beta-galactosidase | (A. niger) |
| beta-glucanase | (B. subtilis, Aspergillus sp.) |
| Glucose-isomerase | (L. brevis, P. notanum, Streptomyces sp.) |
| Glucoseoxidase | (A. niger) |
| Hemicellulase | (A. niger, Trichoderma reesei, Bacillus sp.) |
| Invertase | (S. cerevisae) |
| Catalase | (A. niger) |
| Collagenase | (Clostridium histolyticum) |
| Xsylanase | (A. niger, Trichoderma reesei, Bacillus spp.) |
| Lactase | (S. fragilis, S. lactis, E. coli, Aspergillus sp.) |
| Lipase | (Mould, Yeast) |
| Naringinase | (A. niger) |
| Pancreatin | (Pancreas) |
| Papain | (Papaya) |
| Pectinase | (A. niger, Penicillium sp.) |
| Penicillinamidase | (Bacillus spp.) |
| Penicillinase | (Bacillus spp.) |
| Pepsin | (Animal abdomen) |
| Protease | (A. oryzae, B. subtilis) |
| Pullulanase | (Aerobacter aerogenes) |
| Isoamylase | (Escherichia intermedia, Pseudomonas sp.) |
| Rennin | (Calf stomach, M. miehei, Endothia parasitica) |
| Ribonuclease | (B.subtilis, Mould, A. niger) |
| Cellulase | (A. niger, Trichoderma reesei) |
| Streptokinase | (Streptococcus hemolyticus) |
| Trypsin | (Pancreas) |

### Example 1

### Production of the β-lactamase enzyme of E. coli from the Bacillus subtilis strain

The plasmid pKTH was opened by the Hind III enzyme, and to the cleavage site was joined a gene coding for β-lactamase of E. coli from which the promotor and excretion signal areas had been removed. The hybrid plasmid obtained was transformed into the competent B. subtilis IHO 6140 strain by selecting cells that had received the plasmid, on the basis of their kanamycin resistance, and the cells were cultivated on nutrient medium plates containing kanamycin. The transformants were screened with respect to the yield by suspending each colony in 100 µl 0.1 mM nitrosephin, 0.1 M K-phosphate solution (pH 7.0). Liquid cultures were made of the colonies which gave a positive result in the nitrosephin test (the color of the solution changed into red) for determination of the activity of the β-lactmase enzyme produced. The IHO 6140 B. subtilis strain which had been transformed by the plasmid pKTH 50 was used as control. The strains were grown in a SMS solution (Spizizen minimal salts) to which had been added 0.5 % glycerol, 1 % soluble starch, and 5 µg/ml kanamycin. The cultures were grown at 37°C while shaking. About 5 hours after a logarithmic growth period (Klett₆₇^{ω}250), the cultures were centrifuged at 10,000 xg for 5 minutes and the supernatant was recovered. The cells were suspended in 0.1 M potassium phosphate buffer (pH 2.0) to their original growing volume. The β-lactamase activity was determined in the cell and supernatant fractions by following spectrophotometrically the disintegration of cephalotin. The following results were obtained from the determination.

| | β-lactamase activity(U/ml)* | |
|---|---|---|
| | cells | supernatant |
| B. subtilis IHO 6140/pKTH 50 β-lactamase | 60 | 3000 |
| B. subtilis IHO 6140/pKTH 50 | <10 | <10 |

| | | |
|---|---|---|
| *) 1U of β-lactamase disintegrates 1 µmol penicillin G in 1 minute at 37°C. | | |

### Example 2

### Production of leukocyte interferon in the Bacillus subtilis strain

The plasmid pKTH 53 was cleaved by the Hind III enzyme, and to the cleavage site was joined the DNA sequence coding for the leukocyte interferon (α-2) from which the part coding for the excretion signal had been removed. The obtained hybrid plasmid was transformed into the competent IHO 6140 B. subtilis strain by selecting the cells that had obtained the plasmid, on the basis of their kanamycin resistance. The transformants were screened by a colony hybridization method (Grünstein, M. and Hogness, D.S., Proc. Natl. Acad. Sci. (US) 72:3961-3965 (1975)) while using as a probe the DNA coding for the interferon, marked ¹²⁵J. The bacterium colonies containing interferon-DNA were grown in Luria broth to which had been added 2 % soluble starch and 5 µg/ml kanamycin, while shaking at 37°C. The culture was centrifuged 4 hours after the logarithmic growth period (Klett₆₇^{ω}300) at 10,000 xg for 5 min. The supernatant was recovered, and the cells were suspended to their original growing volume in a 0.9 % NaCl solution. The interferon activity was determined in the cell and supernatant fractions. The B. subtilis IHO 6140/pKTH 53 strain was used as control in the determinations. The following results were obtained from the determinations.

| | Interferon activity (I.U./ml) | |
|---|---|---|
| | cells | supernatant |
| B. subtilis IHO 6140/ pKTH 53-IF | 3000 | 200,000 |
| B. subtilis IHO 6140/ pKTH 53 | <20 | <20 |

### Example 3

### Expression Pertussis Toxin Subunits Inside Bacillus Subtilis

From Bordetella pertussis strain Tohama a 4.5 kb EcoRI-BamHI chromosomal DNA restriction enzyme fragment, containing the pertussis toxin operon, was cloned, utilizing the previously published DNA sequence (Locht, C. et al., Science 232:1258-1264 (1986)). Using Amersham's in vitro oligo-directed mutagenesis system, a HindIII restriction enzyme site was created at the 5' end of the DNA sequence coding for the mature part of pertussis toxin subunits S₁, S₂, S₄ and S₅, as described in United States Patent No 5 010 015 (US application Serial Number 07/308,860) filed on the same date as the present application. The HindIII site in the gene coding for S₃ was created with an oligolinker ligated into the FoKI site positioned immediately downstream of the 5' end of the DNA sequence coding for the mature part of S₃.

To express the pertussis toxin subunits, HindIII fragments containing the coding regions for the subunits were ligated with HindIII cut expression vectors. In this way, the transcription of the subunits genes could be controlled by the amylase promoter (Palva, I., et al., Gene 15:43-51 (1981)) in the vector. The procedure for expressing S₄ was different. The HIndIII fragments coding for S₄ were subjected to a fill-in reaction with Klenow fragment, and the expression vector, to be ligated with the S₄ coding fragment, was digested with EcoRI, followed by a partial S₁ exonuclease digestion and a fill-in reaction with Klenow fragment. Bacillus subtilis strain BRB41 (Palva, I., et al., Gene 22:229-235 (1983)) was transformed with the ligation mixes. The ability of the clones to express the pertussis toxin subunits was analyzed by SDS-polyacrylamide gel electrophoresis, followed by Western blotting. A rabbit antiserum recognizing all subunits and the Protoblot immunoscreening system was used to visualize the immunoreactive proteins. All subunits could be expressed inside Bacillus subtilis.

### Example 4

### Expression of chloramphenicol acetyl transferase (cat) gene in B. subtilis

The chloramphenicol acetyl transferase (cat) gene from B. pumilus is available as a promoterless derivative in the plasmid pPL603 (Williams et al., J. Bacteriol. 146: 1162-1165 (1981)). The AhaIII restriction endonuclease cleaves pPL603 precisely at the 5' end of the cat gene, resulting in a 700 bp AhaIII-XbaI fragment containing the structural cat gene.

To clone this gene in B. subtilis, the plasmid pKTH 39 was linearized with EcoRI, treated with S1 nuclease and Klenow polymerase to generate blunt ends, and subsequently ligated to the corresponding 700 bp AhaIII-XbaI fragment containing the cat gene.

The resulting ligation mixture was transformed into competent B. subtilis BRB 41 cells. Chloramphenicol-resistant (Cm^{r}, 5 µg/ml) colonies were selected, and plasmid DNA from two independent Cm^{r} colonies was analyzed to confirm the correct DNA structure. The results of CAT assay performed from liquid cultures (as described in Shaw, Meth. Enzymol. 43: 737-755 (1975)) were as follows:

| Turbidity (Klett₆₇) | BRB41 (pKTH 39-cat) | | BRB41 | |
|---|---|---|---|---|
| | cells | sup | cells | sup |
| 160 | 0.92 | nr⁻ | nr | nr |
| 250 | 4.7 | nr | nr | nr |
| (Klett₁₀₀ + 5h) | | | | |
| ⁻nr, no results: below detection limit of assay. | | | | |

The structure of the plasmld pKTH 39 containing the cat gene at the region of the ligation of the cat gene to the truncated non-functional α-amylase signal sequence was as follows:

. . . ATG ATT CAA AAA CGA AAG CGG AAT TTA AGC TT . . .
truncated a-amylase signal sequence | cat structural gene

### Example 5

Production of pertussis toxin S4 subunit in B. subtilis Expression of the pertussis toxin S4 subunit in B. subtilis is described in Example 4 of US Patent No. 5 010 015 (U.S. application Serial No. 07/308,860), supra, using the pKTH 39-based expression plasmid pKTH 229. According to this construction, a hybrid S4 subunit is generated containing additional amino acid residues from the α-amylase vector, and lacking several N-terminus amino acids of the S4 subunit.

The subunit was expressed according to the present invention as follows. A plasmid construction was designed to leave only the initiation methionine at the N-terminus of the intact 54 polypeptide. The necessary modifications to the DNA were made employing the well-known polymerase chain reaction (PCR) method, using the plasmid pKTH 229 as a template. The PCR fragment had the following nucleotide sequence at the joint region:

. . .     f.met asp val pro tyr val     . . .
. . .       ATG GAC GTT CCT TAT GTG     . . .
truncated a-amylase     intact S4 subunit sequence
signal sequence

The resulting PCR fragment was ligated to the plasmid pUB110 and transformed into B. subtilis BRB41 cells. Western blotting revealed colonies producing the S4 subunit intracellularly.

## Claims

1. A recombinant DNA molecule comprising:-
(a) the regulation sequence of the α-amylase gene of Bacillus amyloliquefaciens;
(b) DNA encoding a non-functional portion of the signal sequence of the α-amylase gene of Bacillus amyloliquefaciens, said signal sequence portion selected from sequences comprising at least the N-terminal initiation methionine (Met) and the next six adjacent N-terminal amino acids of the wild type of said signal sequence but excluding sequences lacking up to the C-terminal last six consecutive amino acids.
(c) DNA encoding a desired homologous or heterologous amino acid sequence;
wherein (c) is in phase with (a) and (b) and said signal sequence portion does not function to secrete said desired amino acid sequence, with the result that the product of expression of (c) is located intracellularly.

2. A recombinant DNA molecule of claim 1, wherein said non-functional portion of the signal sequence comprises amino acids encoded by ATG ATT CAA AAA CGA AAG CGG.

3. A recombinant DNA molecule of claim 1, wherein said non-functional portion of the signal sequence comprises amino acids encoded by ATG ATT CAA AAA CGA AAG CGG AAT TCC.

4. A recombinant DNA molecule of claim 1, wherein said non-functional portion of the signal sequence comprises amino acids encoded by ATG ATT CAA AAA CGA AAG CGG AAT TCG GAA GCT T.

5. A recombinant molecule of claim 1, wherein said non-functional portion of the signal sequence comprises amino acids encoded by ATG ATT CAA AAA CGA AAG CGG AAT TTA AGC TT.

6. A recombinant DNA molecule of claim 1 wherein said DNA encoding amino acids of a desired protein or polypeptide is DNA encoding chloramphenicol acetyl transferase or DNA encoding pertussis toxin subunits.

7. A vector comprising a recombinant DNA molecule of any of claims 1, 2, 3, 4, 5, or 6.

8. A method for obtaining intracellular expression of a desired homologous or heterologous protein, or polypeptide, in a Bacillus host, comprising
(a) transforming a desired Bacillus host with a recombinant DNA molecule comprising (1) the regulation sequence of the α-amylase gene of Bacillus amyloliquefaciens, (2) DNA encoding a non-functional signal sequence of the α-amylase gene of Bacillus amyloliquefaciens wherein said DNA comprises DNA encoding at least the N-terminal initiation methionine (Met) and the next six adjacent N-terminal amino acids of the wild type of said signal sequence, but not DNA encoding the seven C-terminal amino acids of the wild type of said signal sequence, and (3) DNA encoding amino acids of a desired protein or polypeptide wherein said DNA encoding said desired protein or polypeptide is downstream from and in phase with said regulation sequence and said DNA encoding said non-functional signal sequence, and
(b) culturing the Bacillus host of step (a) under conditions allowing intracellular expression of said desired homologous or heterologous protein or polypeptide.

9. Use of a vector as defined in claim 7 for obtaining intracellular expression of a desired homologous or heterologous protein, polypeptide, or peptide in a Bacillus host.

10. A Bacillus host incorporating a vector of claim 7.

11. Use of a Bacillus host of claim 10 for obtaining intracellular expression of a desired homologous or heterologous protein, polypeptide, or peptide.

## Patentansprüche

1. Ein rekombinantes DNS-Molekül, umfassend:
(a) die Regulationssequenz des α-Amylase-Gens von *Bacillus amyloliquefaciens,*
(b) DNA, kodierend einen nicht-funktionalen Bereich der Signalsequenz des α-Amylase-Gens von *Bacillus amyloliquefaciens,* wobei der Signalsequenzbereich ausgewählt ist aus Sequenzen, umfassend mindestens das N-terminale Initiations-Methionin (Met) und die anschließenden sechs benachbarten N-terminalen Aminosäuren des Wildtyps der Signalsequenz, wobei die Sequenzen bis zu den C-terminalen letzten sechs aufeinanderfolgenden Aminosäuren fehlen,
(c) DNA, kodierend eine gewünschte homologe oder heterologe Aminosäuresequenz,
wobei (c) mit (a) und (b) in Phase ist und der Signalsequenzbereich nicht dazu führt, daß die gewünschte Aminosäuresequenz sezerniert wird, mit dem Ergebnis, daß das Expressionsprodukt von (c) intrazellulär lokalisiert ist.

2. Rekombinantes DNA-Molekül nach Anspruch 1, wobei der nicht-funktionale Bereich der Signalsequenz die Aminosäuren kodiert durch ATG ATT CAA AAA CGA AAG CGG umfaßt.

3. Rekombinantes DNA-Molekül nach Anspruch 1, wobei der nicht-funktionale Bereich der Signalsequenz die Aminosäuren kodiert durch ATG ATT CAA AAA CGA AAG CGG AAT TCC umfaßt.

4. Rekombinantes DNA-Molekül nach Anspruch 1, wobei der nicht-funktionale Bereich der Signalsequenz die Aminosäuren kodiert durch ATG ATT CAA AAA CGA AAG CGG AAT TCG GAA GCT T umfaßt.

5. Rekombinantes Molekül nach Anspruch 1, wobei der nicht-funktionale Bereich der Signalsequenz die Aminosäure kodiert durch ATG ATT CAA AAA CGA AAG CGG AAT TTA AGC TT umfaßt.

6. Rekombinantes DNA-Molekül nach Anspruch 1, wobei die DNA, kodierend Aminosäuren eines gewünschten Proteins oder Polypeptids, DNA ist, kodierend Chloramphenicolacetyl-Transferase oder DNA, kodierend Pertussistoxin-Untereinheiten.

7. Ein Vektor, umfassend ein rekombinantes DNA-Molekül gemäß einem der Ansprüche 1 bis 6.

8. Ein Verfahren zum Erhalten intrazellulärer Expression eines gewünschten homologen oder heterologen Proteins oder Polypeptids in einem *Bacillus*-Wirt, umfassend
(a) Transformieren eines gewünschten *Bacillus*-Wirts mit einem rekombinanten DNA-Molekül, umfassend (1) die Regulationssequenz des α-Amylase-Gens von *Bacillus amyloliquefaciens,* (2) DNA, kodierend eine nicht-funktionale Signalsequenz des α-Amylase-Gens von *Bacillus amyloliquefaciens,* wobei die DNA DNA, kodierend mindestens das N-terminale Initiations-Methionin (Met) und die anschließenden benachbarten sechs N-terminalen Aminosäuren des Wildtyps der Signalsequenz umfaßt, aber nicht DNA, kodierend die sieben C-terminalen Aminosäuren des Wildtyps der Signalsequenz, und (3) DNA, kodierend Aminosäuren eines gewünschten Proteins oder Polypeptids, wobei die DNA, kodierend das gewünschte Protein oder Polypeptid stromabwärts von und in Phase mit der Regulationssequenz ist, und die DNA, kodierend die nicht-funktionale Signalsequenz, und
(b) Kultivieren des *Bacillus*-Wirts aus Schritt (a) unter Bedingungen, die die intrazuelluläre Expression des gewünschten homologen oder heterologen Proteins oder Polypeptids erlaubt.

9. Verwendung eines Vektors nach Anspruch 7 zum Erhalten intrazellulärer Expression eines gewünschten homologen oder heterologen Proteins, Polypeptids oder Peptids in einem *Bacillus*-Wirts.

10. Ein *Bacillus*-Wirt, enthaltend einen Vektor nach Anspruch 7.

11. Verwendung eines *Bacillus*-Wirts nach Anspruch 10 zum Erhalten intrazellulärer Expression eines gewünschten homologen oder heterologen Proteins, Polypeptids oder Peptids.

## Revendications

1. Molécule d'ADN recombinant comprenant:
(a) la séquence de régulation du gène de l'α-amylase de Bacillus amyloliquefaciens;
(b) l'ADN codant pour une partie non fonctionnelle de la séquence signal du gène de l'α-amylase de Bacillus amyloliquefaciens, ladite partie de la séquence signal choisie parmi des séquences comprenant au moins la méthionine (Met) d'initiation de l'extrémité N et les six acides aminés suivants adjacents de l'extrémité N du type sauvage de ladite séquence signal mais excluant les séquences auxquelles il manque jusqu'aux six derniers acides aminés consécutifs à l'extrémité C;
(c) l'ADN codant pour une séquence d'acides aminés homologue ou hétérologue désirée;
où (c) est en phase avec (a) et (b) et ladite partie de la séquence signal ne dirige pas la sécrétion de ladite séquence désirée d'acides aminés, ce qui a pour résultat que le produit d'expression de (c) est localisé à l'intérieur de la cellule.

2. Molécule d'ADN recombinant selon la revendication 1, dans laquelle ladite partie non fonctionnelle de la séquence signal comprend les acides aminés codés par ATG ATT CAA AAA CGA AAG CGG.

3. Molécule d'ADN recombinant selon la revendication 1, dans laquelle ladite partie non fonctionnelle de la séquence signal comprend les acides aminés codés par ATG ATT CAA AAA CGA AAG CGG AAT TCC.

4. Molécule d'ADN recombinant selon la revendication 1, dans laquelle ladite partie non fonctionnelle de la séquence signal comprend les acides aminés codés par ATG ATT CAA AAA CGA AAG CGG AAT TCG GAA GCT T.

5. Molécule d'ADN recombinant selon la revendication 1, dans laquelle ladite partie non fonctionnelle de la séquence signal comprend les acides aminés codés par ATG ATT CAA AAA CGA AAG CGG AAT TTA AGC TT.

6. Molécule d'ADN recombinant selon la revendication 1 dans laquelle ladite molécule d'ADN codant pour la séquence de la protéine ou du polypeptide désiré est une molécule d'ADN codant pour la chloramphénicol acétyl transférase ou l'ADN codant pour des sous-unités de la toxine coquelucheuse.

7. Vecteur comprenant une molécule d'ADN recombinant selon l'une des revendications 1, 2, 3, 4, 5 ou 6.

8. Procédé permettant d'obtenir une expression intracellulaire d'une protéine ou d'un polypeptide homologue ou hétérologue désiré dans un hôte bactérien Bacillus comprenant
(a) la transformation d'un hôte bactérien Bacillus désiré avec une molécule d'ADN recombinant comprenant (1) la séquence de régulation du gêne de l'α-amylase de Bacillus amyloliquefaciens; (2) l'ADN codant pour une séquence signal non fonctionnelle du gène de l'a-amylase de Bacillus amyloliquefaciens, ladite séquence d'ADN comprenant au moins la méthionine (Met) d'initiation de l'extrémité N et les six acides aminés suivants adjacents à l'extrémité N du type sauvage de ladite séquence signal mais non l'ADN codant pour les sept acides aminés de l'extrémité C du type sauvage de ladite séquence signal, et (3) l'ADN codant pour les acides aminés d'une protéine ou d'un polypeptide désiré où ledit ADN codant pour ladite protéine ou ledit polypeptide désiré se trouve en aval de ladite séquence de régulation et de ladite séquence signal non fonctionnelle et en phase avec ces séquences, et
(b) la culture de l'hôte Bacillus de l'étape (a) dans des conditions permettant l'expression intracellulaire de ladite protéine ou dudit polypeptide homologue ou hétérologue désiré.

9. Utilisation d'un vecteur comme défini à la revendication 7 pour obtenir une expression intracellulaire d'une protéine, d'un polypeptide ou d'un peptide, homologue ou hétérologue, dans un hôte Bacillus.

10. Hôte Bacillus incorporant un vecteur selon la revendication 7.

11. Utilisation d'un hôte Bacillus selon la revendication 10 pour obtenir une expression intracellulaire d'une protéine, d'un polypeptide ou d'un peptide, homologue ou hétérologue désiré.
